# EUROPEAN PATENT SPECIFICATION

(11) **EP 0 355 036 B1**
(45) Date of publication and mention of the grant of the patent: **13.02.2002**
(21) Application number: 89201454.9
(22) Date of filing: 06.06.1989
(51) Int. Cl.: C12N 15/52, C12N 1/21

(54) **Process for selecting and maintaining recombinant DNA in lactic acid bacteria**
Verfahren zur Selektion und zur stabilen Einschleusung rekombinanter DNA in Milchsäurebakterien
Procédé pour sélectionner et maintenir l'ADN recombinant dans les bactéries lactiques

(30) Priority: 15.06.1988 NL 8801529
(43) Date of publication of application: 21.02.1990
(73) Proprietor: NEDERLANDS INSTITUUT VOOR ZUIVELONDERZOEK, 6710 BA Ede (Gld) (NL)
(72) Inventor: De Vos, Willem Meindert, NL-6714 MD Ede (Gld.) (NL)
(74) Representative: de Bruijn, Leendert C.

(56) References cited:
- EP-A- 0 228 726
- WO-A-85/03945
- NETHERLANDS MILK AND DAIRY JOURNAL, vol. 40, no. 2/3, 1986, pages 141-154, Wageningen, NL; W.M. DE VOS: "Gene cloning in lactic streptococci"
- FOOD TECHNOLOGY, vol. 40, no. 10, October 1986, pages 95-98, Institute of Food Technologists, Chicago, Illinois, US; C.A. BATT: "Genetic engineering of lactobacillus"
- JOURNAL OF BACTERIOLOGY, vol. 167, no. 3, September 1986, pages 855-862, American Society for Microbiology, Washington, DC, US; J.M. INAMINE et al.: "Molecular and genetic characterization of lactose-metabolic genes of Streptococcus cremoris"
- APPLIED AND ENVIRONMENTAL MICROBIOLOGY, vol. 48, no. 2, August 1984, pages 347-351, American Society for Microbiology, Washington, DC, US; S.K. HARLANDER et al.: "Molecular cloning of the lactose-metabolizing genes from streptococcus lactis"
- BIOCHIMIE, vol. 70, April 1988, pages 523-529, Société de Chimie biologique, Elsevier, Paris, FR; M. SHIMIZU-KADOTA: "Cloning and expression of the phospho-beta-galactosidase genes on the lactose plasmid and the chromosome of Lactobacillus casei C257 in Escherichia coli"
- BIOCHIMIE, vol. 70, April 1988, pages 461-473, Société de Chimie biologique, Elsevier, Paris, FR; W.M. DE VOS et al.: "Molecular cloning of lactose genes in dairy lactic streptococci: the phospho-beta-galactosidase and beta-galactosidase genes and their expression products"
- GENE, vol. 62, 15th March 1988, pages 249-261, Elsevier Science Publishers B.V. (Biomedical Division), Amsterdam, NL; B. BOIZET et al.: "Isolation and structural analysis of the phospho-beta-galactosidase gene from Streptococcus lactis Z268"
- GENE, vol. 62, 15th March 1988, pages 277-288, Elsevier Science Publishers B.V. (Biomedical Division), Amsterdam, NL; C.-A. ALPERT et al.: "Molecular cloning and nucleotide sequence of the factor IIIlac gene of Lactobacillus casei"
- ANTONIE VAN LEEUWENHOEK, vol. 49, 1983, pages 259-274, Wageningen, NL; L.L. McKAY: "Functional properties of plasmids in lactic streptococci"

## Description

The invention relates to a procedure for selecting lactic acid bacteria which contain recombinant DNA, and keeping said DNA stable, with the aid of a marker acceptable in foodstuffs.

The development of various host-vector systems makes it possible to use recombinant DNA techniques in improving the characteristics of lactic acid bacteria which are used in a large number of industrial dairy fermentations and other foodstuff fermentations (see FEMS Microbiol. Rev. 46 (1987), 281-325, FEMS Microbiol. Letters 44 (1987), 173-177). A number of improvements in which use is made of homologous and heterologous genes have already been described (see EP-A 01,574,414, EP-A-0,228,726, Dutch Patent Application 87.01378). Indispensable to these techniques are selection markers which are used to select the bacterium which has been modified in the desired manner. In addition, in many cases it is necessary to exert selection pressure on the genetically modified bacterium obtained in order to prevent the desired modifications being lost. This latter applies in particular, but not exclusively, if the recombinant DNA is a constituent of a recombinant plasmid which is capable of autonomous replication in the lactic acid bacterium used.

International patent application WO 85/03945 discloses a procedure for selecting S.lactis (Lactococcus lactis subsp. lactis), which contain recombinant DNA, in which said S.lactis, which lacks one or two proteinases, is transformed with a DNA fragment comprising the BclIB fragment of pLP712. This fragment, which comprises many genes of the lactose-metabolism -in any case the genes for Factor III, Enzyme II and phospho-β-galactosidase- has the size of approximately 12 kb.

In view of this, WO 85/03945 does not disclose a practical solution for using the lactose metabolism as a marker. Instead, a recombinant plasmid (pGKV500) which confers erythromycin resistance and which encodes a Prt⁺ phenotype is used as a marker. However, the Prt⁺ phenotype cannot be used as a suitable marker for selecting transformance on agar.

The use of genes indispensible for the lactose metabolism (in particular lactose specific Factor III and phosphor-β-galactosidase) as an auxotrophic marker for transforming S.sanguis is described in J.M.Inamine et al., Journal of Bacteriology, Vol. 167, No.3, pages 855-862. However, S.sanguis is not suitable for use in foodstuffs. Furthermore, the *Lac*-genes are integrated through recombination into the S.sanguis chromosomal DNA; the gene to be introduced is not incorporated in the chromosome of S.sanguis. Therefore, according to this reference, the marker is incorporated, but not the gene or recombinant DNA which is to be maintained in a stable manner.

An ideal selection marker satisfies, inter alia, the following criteria: (i) it provides the possibility of simple and dominant selection; (ii) it is composed of well-defined, preferably homologous DNA; (iii) it presents the possibity of being used on an industrial scale; and (iv) if it is used in lactic acid bacteria intended for, for example, human consumption, it is completely foodstuffs-safe.

Hitherto, only antibiotic-resistant markers, which satisfy merely the first of the above stated criteria, have been used in lactic acid bacteria. Major drawbacks in the use of said markers are the high costs and the fact that one or more antibiotics must always be present in the medium in which the bacteria are cultivated, and this makes a direct application in the foodstuffs industry impossible.

Therefore there is a considerable need for so-called foodstuffs-safe selection markers which can be used in the construction of genetically modified lactic acid bacteria which can be applied in industrial processes.

Some foodstuffs-safe selection markers which could be used in lactic acid bacteria have been described, such as bacteriophage-resistance, bacteriocin-resistance and the ability to produce proteinase. These markers are, however, unsuitable for use in industrial processes because bacteriophages are undesirable under practical conditions, the addition of bacteriocins is a costly business which is not always legally permitted, and proteinase-producing lactic acid bacteria cannot be recognized in a simple way and, in addition, are rapidly overgrown by mutants which do not produce any proteinase.

In addition, the possibility exists for using so-called auxotrophic mutants which, in contrast to the so-called prototrophic, wild-type strains, are incapable of optimum growth in media without supplementation of nutrients. The genetic information for the synthesis of such nutrient(s) can be obtained by recombinant DNA techniques and be used together with or without a replicon, but preferably in conjunction with gene(s) which give rise to the synthesis of desired proteins and/or characteristics as a means of maintaining selection pressure on the genetically modified bacterium.

Because most wild-type lactic acid bacteria already need a large number of nutrients for optimum growth, it is not simple to isolate such auxotrophic, mutant lactic acid bacteria. In industrial processes media are used in which lactic acid bacteria grow optimally and which are therefore already very rich in a large number of nutrients, which hampers a direct selection pressure to a considerable extent. A solution to comparable problems in Bacillus is presented by making use of a host which is deficient in an element of the synthesis or maintenance of the cell envelope, in this case D-alanine, in combination with an extrachromosomal element which is able to suppress this requirement, in this case coding for the D,L-alanine racemase (EP-A-O,185,512). There is, however, the question of what the significance is of D-alanine for the integrity of the cell envelope of lactic acid bacteria, of whether lactic acid bacteria have a D,L-alanine racemase, of whether mutants can be obtained which are disturbed in the synthesis of D,L-alanine racemase, and/or of whether the autosynthesis of D-alanine is necessary in growth on the complex media which are used for cultivating lactic acid bacteria.

An example of an auxotrophic marker to which these limitations do not apply is the ability to ferment lactose. Because lactose is the only fermentable sugar which is present in milk or media derived from milk, lactic acid bacteria which have lost the ability to use lactose as an energy source (so-called lactose-deficient, lac⁻ mutants) are unable to grow in these media. All the lactic acid bacteria used in the dairy industry therefore have the ability to ferment lactose.

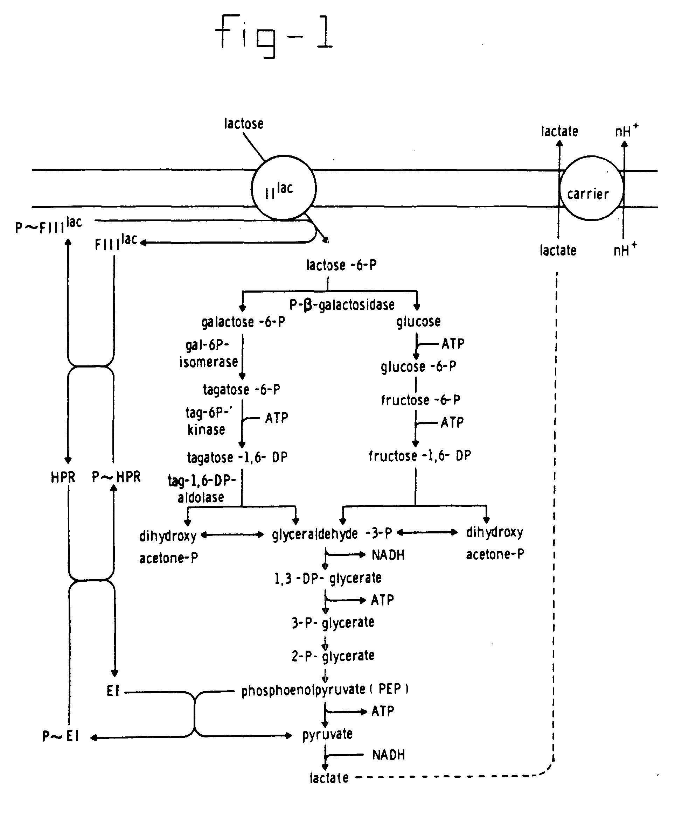

The manner in which this lactose fermentation takes place has been described well in a number of cases. In particular the manner in which the mesophilic lactic acid bacteria Streptococcus lactis and Streptococcus cremoris (recently renamed as Lactococcus lactis subsp. lactis and Lactococcus lactis subsp. cremoris, respectively; Syst. Appl. Microbiol. 6 (1985) 183-195) ferment lactose to form a lactate has been well investigated and is reproduced in Figure 1. From this it must be deduced that the lactose fermentation in said lactic acid bacteria is a complex process which involves at least 19 different reactions catalysed by enzymes.

Genetic research has revealed that, in this group of lactic acid bacteria, the genes coding for the enzymes involved in the lactose fermentation are situated in many cases partly on the bacterial chromosome and partly on the extrachromosomal plasmid DNA. If the plasmid coding for genes which are involved in the lactose fermentation (so-called lactose plasmid) is lost from such a strain, the bacterium is no longer capable of fermenting lactose. Such lac⁻ mutants can be obtained in a simple way by using plasmid-curing methods and can be distinguished in a simple way from a lac⁺ strain (J. Bacteriol. 154 (1983) 1-9; Appl. Microbiol. 23 (1972) 1090-1096).

Lactose plasmids can be transferred from a lac⁺ to lac⁻ strains with the aid of various genetic methods such as conjugation, transduction, protoplast fusion and transformation (Ant. van Leewenhoek 49 (1983), 257-282; Appl. Environ. Microbiol. 48 (1984), 252-259). In plasmid transfer with the aid of transduction, there is the possibility of a lactose plasmid being completely or partially integrated in the chromosomal DNA of the recipient lactic acid bacterium (Appl. Microbiol. 36 (1978), 360-367).

It has been shown that, in L. lactis C2, the genes coding for the lactose-specific enzymes, Enzyme II, Factor III and P-β-galactosidase (P-β-Gal), so-called lactose genes, are situated on an approx. 50 kb plasmid DNA (J. Bacteriol. 102 (1970), 804-809; Appl. Environ. Microbiol. 35 (1978), 592-600). Furthermore, there are indications that, in L. lactis strains, the tagatose genes coding for the enzymes of the tagatose-6P pathway, viz. galactose-6-P isomerase, tagatose-6-P kinase and tagatose-1,6-diP aldolase, are also localized on a lactose plasmid DNA (J. Bacteriol. 153 (1983) 76-83). It is remarkable that the enzymes coded by the lactose and tagatose genes are not indispensable for a lactic acid bacterium because the fermentation of sugars other than lactose (and sometimes galactose), such as glucose, remains possible. The L. lactis genes coding for P-β-Gal and tagatose-1,6-diP aldolase have been cloned in Escherichia coli (J. Gen. Microbiol. 132 (1986), 331-340; FEMS Microbiol. Letters 33 (1986), 79-83), while only the P-β-Gal gene has also been cloned and expressed in L. lactis (EP-A-0,228.726).

It has been shown in L. lactis NCDO 712 that the plasmid-localized genes of the lactose metabolism are possibly situated on an approx. 12 kb restriction fragment (so-called BclIB fragment) derived from the largest 56.5 kb plasmid pLP712 (J. Bacteriol. 154 (1983), 1-9). The same genes are situated on a deletion derivative of pLP712, the lactose miniplasmid pMG820, which nevertheless still has a size of 23.7 kb (J. Gen. Microbiol. 132 (1986), 331-340).

By introducing a plasmid vector incorporating the 12 kb BclIB restriction fragment containing the pLP712 lactose genes into a L. lactis strain from which the lactose plasmid has been removed, the ability to ferment lactose could possibly be restored again. This method is described as possible in BP-A-0,157,441, although no actual example of an implementation is provided. A problem in this connection is the size of the BclIB fragment which not only contains the three lactose genes but probably also three tagatose genes. Because a cloning factor including the selection marker is preferably as small as possible in view of manipulatability and copy number, the use of this large BclIB fragment in practice is not considered likely.

The use of lactose-deficient lactic acid bacteria in which differing from the abovementioned mutants, only one or two of the lactose genes are mutated and which can be complemented with small, well-defined DNA fragments, is therefore to be preferred.

### Description of the Invention

The invention now provides a method for selecting lactic acid bacteria which contain recombinant DNA, and keeping said DNA stable, with the aid of a marker acceptable in foodstuffs for human consumption, characterised in that a lactic acid bacterium which lacks one or two enzymes indispensable for lactose fermentation, chosen from Enzyme II, Factor III, p-β-galactosidase, gal-6-P-isomerase, tagatose-6-P-kinase, tagatose-1,6-diP-aldolase and/or is transformed with a DNA fragment which, as a marker,
- contains one or more genes coding at least for the said one or two enzymes lacking in said lactic acid bacterium and
- has the size of at most the genes coding for phospho-β-galactosidase and enzyme III,
and also contains one or more structural genes coding for an improved or new characteristic for the lactic acid bacterium.

Two components are central in this invention: the lactose deficient lactic acid bacterium and the DNA fragment coding for the one or two aforementioned enzymes from the lactose metabolism.

The lactose-deficient lactic acid bacterium is incapable of fermenting lactose because said lactic acid bacterium either completely lacks one or two of the abovementioned enzymes which are essential for fermenting lactose, or contains said abovementioned enzyme or enzymes with the limitation that said enzyme or said enzymes has or have respectively insufficient enzymatic activity to convert lactose at the required rate. In addition it is desirable that, apart from the inability to ferment lactose, the said lactic acid bacterium does not differ from other lactic acid bacteria which are used in industrial fermentation. The nature of the genetic deficiency may be a deletion, insertion or point mutation in the DNA coding for an enzyme which is involved in the lactose metabolism. Preferably the deficiency involves the lactose or tagatose genes. In particular, the Factor III or P-β-Gal genes are very suitable because it is known that these code for relatively small, biochemically well characterized, intracellular, soluble enzymes.

Said deficiency can be achieved in various ways, such as by conventional random mutagenesis with the aid of irradiation with ultraviolet light or treatment with mutagenic substances such as ethyl methanesulfonate or nitrosoguanidine. Lactose-deficient mutants can also be obtained by means of transposon mutagenesis. Site-directed mutagenesis with the aid of recombinant DNA techniques can be used combined with the transformation systems described. A possibility in this connection is to replace the wild-type gene with the modified lactose gene by means of integration in the DNA of the lactic acid bacterium. If the lactose gene to be mutated is localized on a plasmid, there is the possibility of replacing the wild-type gene with the modified lactose gene in vitro and reintroducing the lactose plasmid thus mutated into a lactic acid bacterium. Preferably, such a manipulation is carried out with a small lactose plasmid. The lactose miniplasmid pMG820 is very suitable for this purpose. In addition, it is possible to integrate a lactose plasmid, either before or after mutagenesis, in the chromosomal DNA of the lactic acid bacterium by making use of transduction. This technique also presents the possibility of transferring integrated lactose genes from one lactic acid bacterium strain to the other.

It is desirable for applications that the lactose-deficient lactic acid bacterium is a stable mutant in which back-mutation to a lactose-proficient phenotype occurs only with a very low frequency.

Preferably a Lactococcus or a Lactobacillus is used as lactic acid bacteria. It is known that bacteria of this type are suitable for use in various industrial fermentations and also that they ferment lactose according to the manner indicated in Figure 1. In addition, recombinant DNA techniques can be used in these lactic acid bacteria. In particular, Lactococcus lactis is very suitable owing to the use of this lactic acid bacterium in a large number of dairy fermentations in which lactose is the only fermentable sugar.

The DNA fragment which is used in the lactose-deficient lactic acid bacteria as a marker has to code for the one or two abovementioned enzymes which are absent from or have insufficient activity in said lactic acid bacterium. This means that said DNA fragment must contain the structural information for these one or two enzymes and also possibly expression signals which are necessary to achieve an expression such that wild-type fermentation of lactose is possible. Such a DNA fragment can be obtained by complementing the said lactose-deficient lactic acid bacterium using host-vector and expression systems developed for lactic acid bacteria and other bacteria. A particular example of this is given in EP-A-0,228,726 which describes the stepwise cloning and expression in L. lactis of the pMG820-coded P-β-Gal gene.

The lactose deficiency of the lactic acid bacterium used determines the number and type of the genes which code for the one or two aforementioned enzymes absent from said lactic acid bacterium and indispensable for the lactose fermentation. Preferably, use is made of the lactose and tagatose genes. In particular, the genes which code for P-β-Gal and Factor III can be used, which are small in size, can be isolated on usable restriction fragments and are able to complement those L. lactis mutants which are deficient in the synthesis of said enzymes.

The said DNA fragment may also code for one or more functions which imparts or impart an improved or novel characteristic or characteristics to the lactic acid bacterium. Examples of this are the structural genes including expression and regulation signals which code for proteinases and peptidases which enable the lactic acid bacterium to decompose protein into peptides and/or amino acids, or for bacteriophage-resistant mechanisms, or for enzymes which affect the decomposition of citrate, or for enzymes involved in bacteriocin production and/or bacteriocin immunity, or code for heterologous proteins such as the lactose-hydrolysing β-galactosidase, the starch-cleaving α-amylase or the milk-curdling chymosin, which may or may not be secreted by the lactic acid bacteria.

In addition, the DNA fragment may contain a replicon which is functional in lactic acid bacteria and which distributes the DNA fragment, if circularized, as an extrachromosomal plasmid over the progeny of the lactic acid bacteria used. Such a DNA fragment may then be used directly or after some improvements which are obvious to anyone knowledgeable of the state of the art, as cloning, expression or secretion vector. Examples of readily usable replicons are those originating from the L. lactis plasmid pSH71 or the conjugative plasmid pAMβ1 which can replicate with a high or with a low copy number in a large number of lactic acid bacteria.

In the complementary case where the DNA fragment does not contain a functional replicon, inheritance can only be achieved if the DNA fragment is completely or partly integrated into a replicon (either a plasmid or the chromosomal DNA) which is already present in the transformed lactic acid bacteria. The efficiency of this occurrence is increased in general if said DNA fragment also has DNA sequences which are homologous with parts of said replicons already present. Integration into the bacterial chromosome also presents the possibility of amplifying the integrated DNA. Selection for this can be carried out in a simple manner if the DNA fragment used as marker, for example through the presence of weak expression signals can only result in wild type expression of the DNA, which codes for the complementary enzymes indispensable for the lactose fermentation, after amplification to a large number of copies.

The combination of said lactic acid bacterium and DNA fragment should finally be chosen in a manner such that actual selection and stable maintenance of the DNA fragment can be carried out if lactose is present as the only fermentable sugar in the culture medium to be used.

### EXPERIMENTAL PART

### Bacterial strains and plasmids used

The E.coli strains JM83 (Gene 19 (1982) 259-268) was used for the routine sub-cloning with pUC vectors. Furthermore, E.coli JM103 (Nucl. Acids Res. 9 (1981), 309-321) was used for M13 cloning and E.coli MC1061 (J. Mol. Biol. 138 (1980) 179-207) for cloning using the vector pAT153 and pNZ12. Strain MG1363 is a plasmid-free derived strain of L.lactis NCDO 712 (J> Bacteriol. 154 (1983) 1-9). Strain MG1820 is a derivative of MG1363 which contains the mini-lactose plasmid pMG820 (J. Gen. Microbiol. 132 (1986) 331-340). Both L.lactis strains originate from M.J. Gasson, AFRC Institute of Food Research, Norwich, U.K.). L.lactis Y2-5 is a Factor III-deficient mutant obtained by ethyl methanesulfonate mutagenesis of strain YP2. L.lactis YP2 is a lactose-constitutive derivative of L.lactis C2 in which the plasmid-coded genes coding for the lactose metabolism are integrated into the chromosomal DNA with the aid of transduction (J. Bacteriol. 149 (1982) 420-427, J. Dairy Sc. 67 (1984), 950-959). L.lactis Y2-5 originates from L.L. McKay, University of Minnesota, St. Paul, USA.

The E.coli plasmid vectors pUC7 and pUC18/19 have a size of approx. 2.7 kb and code for ampicillin resistance (ApR) (Gene 19 (1982), 259-268; Gene 33 (1985), 103-119). The E.coli vector pAT153 (Nature 283 (1980), 216-218) is approx. 3.7 kb and codes for ApR and also for tetracyclin resistance (TcR). The M13 vectors Mp10 and Mp11 have been described (Nucl. Acids Res. 9 (1981), 309-321).

The L.lactis plasmid vector pIL253(Biochimie 70 (1988), parts 3 and 4) has a size of approx. 4.8 kb, codes for erythromycin resistance (Emr) and is a high copy-number derivative of the conjugative plasmid pAMβ1 (Appl. Environ. Microbiol. 52 (1986), 394-399) and originates from A. Chopin, INRA, Jouy-en-Josas, France. The construction of the 4.3 kb plasmid vector pNZ12 replicating, inter alia, in E.coli and lactic acid bacteria and coding for resistance to chloramphenicol (CmR) and kanamycin (KmR), has been described, as have pNZ32 (7.5 kb) and pNZ367 (8.3 kb) which both contain the L.lactis pMG820 P-β-Gal, cloned in pNZ12 (EP-A-0,228,726).

### Manipulation of E. coli, S. lactis and DNA

All the manipulations with E.coli and of DNA in vitro were carried out according to standard procedures (Molecular Cloning, A Laboratory Manual, Cold Spring Harbor 1982) and/or as described by the suppliers of the restriction and other DNA-modifying enzymes (Bethesda Research Laboratories and Boehringer). Specific techniques for L.lactis, such as cultivation and media, plasmid isolation and protoplast transformation, have been described (Appl. Environ. Microbiol. 48 (1984), 252-259); EP-A-0,228,726). M17 medium (Difco) supplemented with 0.5% lactose (LM17) or 0.5% glucose (GM17) were routinely used for cultivating L.lactis. In the stability experiments involving L.lactis, use was also made of a whey permeate (WP) medium which is composed of 5% ultrafiltered and dried whey containing 1.9% β-glycerophosphate, 0.05% yeast extract and 0.05% casein hydrolysate. This WP medium, which can be used in the dairy industry, contains lactose as the only fermentable sugar. Lactose-indicator agar (LIA) containing Elliker broth, lactose and the pH indicator bromocresol purple was used for the selection of lac⁺ and lac⁻ colonies as described (Appl. Microbiol. 23 (1972), 1090-1096). Complete cells of L.lactis, precultivated in M17 medium containing 40 mM D,L-threonine were transformed with the aid of a Gene Pulser Electroporator in electroporation buffer consisting of 25% sucrose, 1 M MgCl₂ and 5 mM potassium phosphate buffer, pH 7.0, essentially according to the instructions of the supplier (Biorad) with a single electrical pulse of 6250 V/cm and a capacitance of 25 uF.

### Determination of DNA nucleotide sequence and DNA synthesis

Suitable restriction fragments were cloned with the aid of M13 vectors cloned in JM103 (Nucl. Acids Res. 9 (1981), 309-321) and the nucleotide sequence was determined with the dideoxy chain termination method (Proc. Natl. Acad. Sci. 74 (1977), 5463-5467). DNA oligonucleotides to be used as a primer for the DNA sequence determination or for the construction of mutations were synthesised according to the phosphoramidite method with the aid of a cyclone DNA synthesiser according to the manufacturer's instructions (New Brunswick Scientific).

### Protein analysis

Proteins were analyzed with the aid of SDS polyacrylamide gel electrophoresis (Nature 227 (1970), 680-685), followed by dyeing as in relevant cases with Coomassie blue. Determination of the NH₂ terminus of blotted protein was carried out with the aid of a gas-phase sequencer (Applied Biosystems) as described (Eur. J. Biochem. 152 (1986), 9-19). Immunoblotting was carried out with the aid of rabbit antibodies in combination with peroxidase-labelled goat anti-rabbit antibodies as described by the supplier (Bethesda Research Laboratories). The activity of the P-β-Gal was determined as described (J. Gen. Microbiol. 132 (1986), 331-340).

### EXAMPLES

### Example 1

Identification, characterization and cloning of the S. lactis pMG820-coded lactose genes.

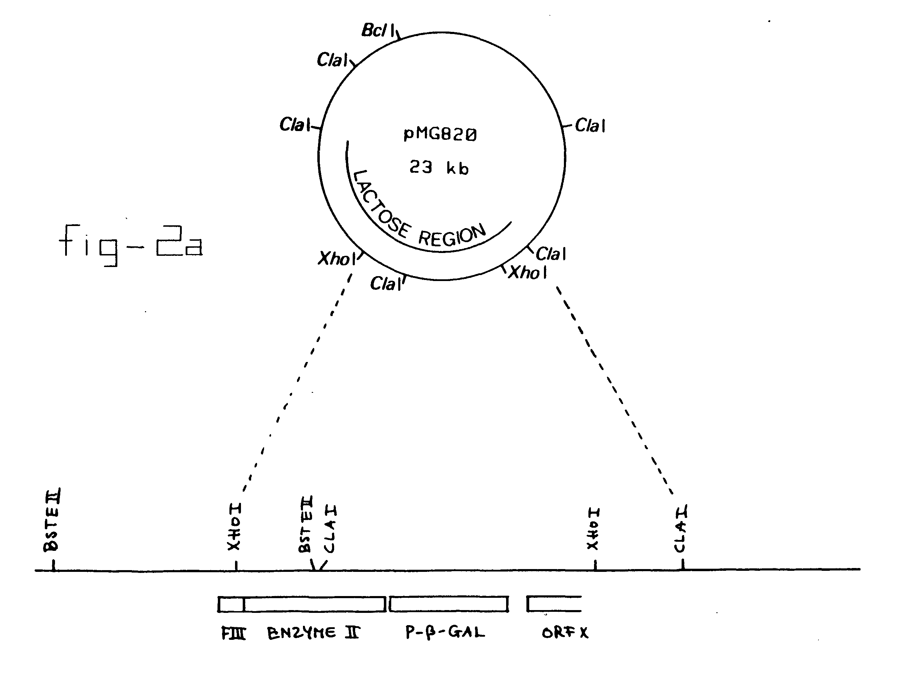

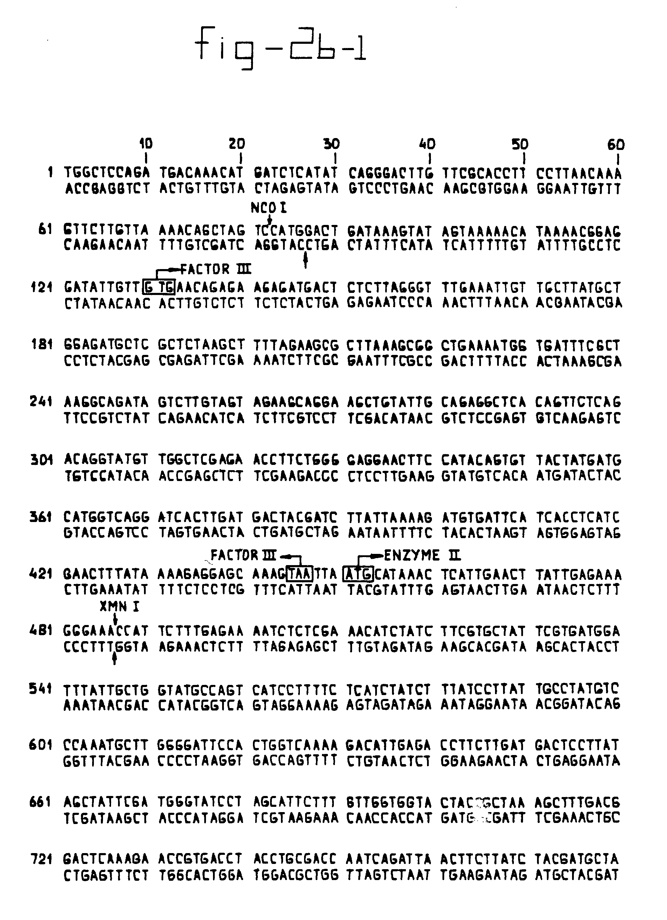

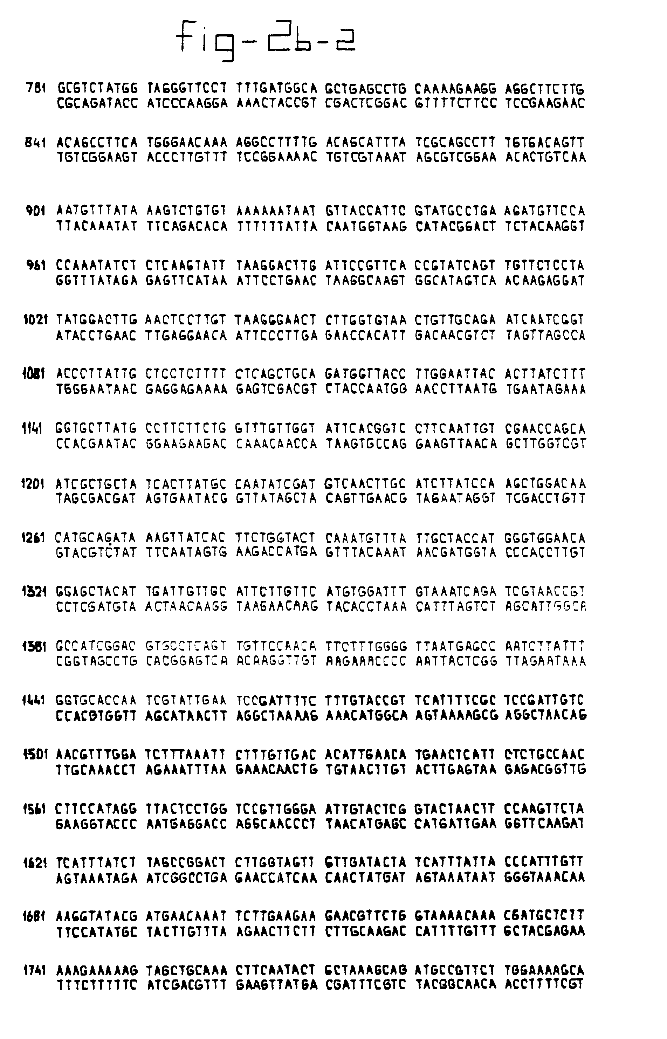

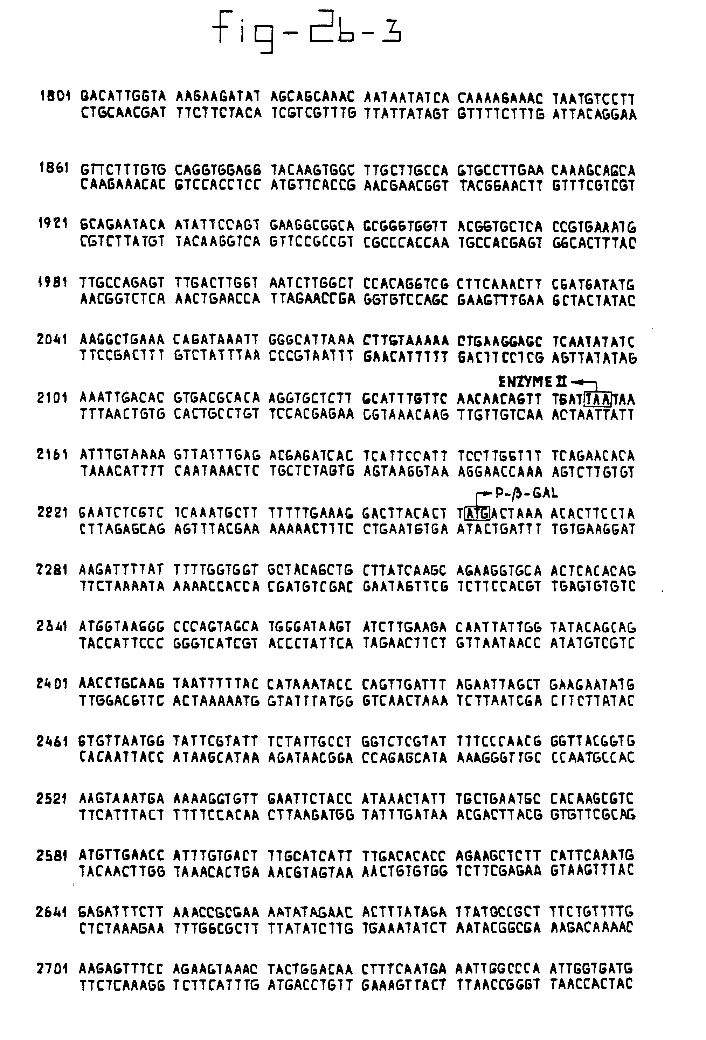

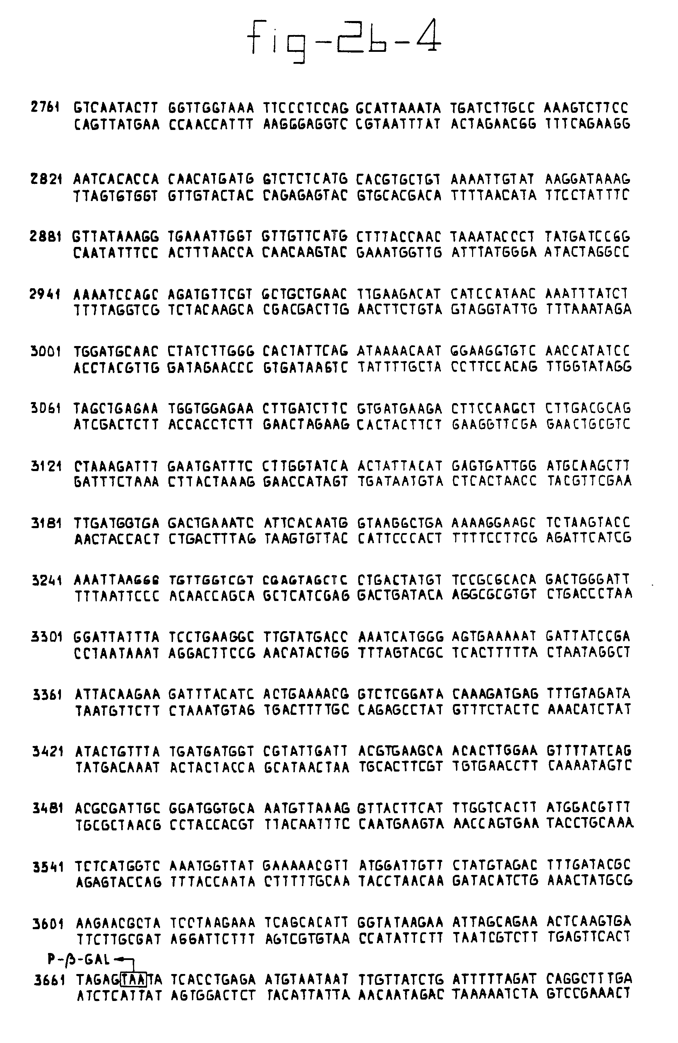

The 4.4 kb XhoI fragment of the L.lactis mini lactose plasmid pMG820 contains the P-β-Gal gene (J. Gen. Microbiol. 132 (1986), 331-340; see Figure 2A). The DNA sequence of this gene and about 2 kb of the upstream DNA have been determined and are shown in Figure 2B. In addition to an open reading frame in the position expected for the P-β-Gal gene, there are two open reading frames present in this upstream region. The following experiments support the conclusion that the first open reading frame (129-448) codes for the Factor III lactose, the second open reading frame (450-1708) codes for Enzyme II lactose, and the third open reading frame (2262-1407) codes for the P-β-Gal:
(i) the NH₂ termini of purified Factor III and P-β-Gal correspond to those derived from the DNA sequence of the first and last open reading frames;
(ii) when cloned in E.coli, a DNA fragment containing the first open reading frame gives rise to the synthesis of a protein having a sub-unit molecular weight of approx. 10 kD, which reacts with anti-Factor III antibodies;
(iii) when cloned in E.coli, a DNA fragment containing the third open reading frame gives rise to the synthesis of an approx. 60 kD protein which has P-β-Gal activity;
(iv) when analyzed in a so-called hydrophobicity plot (J. Mol. Biol. 157 (1982), 105-132), the amino acid sequence derived from the DNA sequence of the second open reading frame gives the transmembrane domains which are expected for an integral membrane protein such as Enzyme II;
(v) both the nucleotide sequence of the DNA in all three open reading frames and also the derived amino acid sequence exhibit a high degree of homology with those of Factor III, Enzyme II and P-β-Gal of Staphyloccus aureus (J. Biol. Chem. 262 (1987), 16444-16449).

The lactose genes are followed by a possible intercistronic region of 231 nucleotides, after which a new open reading frame of no less than 700 nucleotides starts at position 3959 and probably codes for the 50 kD protein X, the existence of which has been demonstrated previously (J. Gen. Microbiol. 132 (1986), 331-340) and which possibly plays a part in the expression of the lactose genes.

### Example II

Complementation of an L.lactis Y2-5 by a DNA fragment containing the pMG820 Factor III gene.

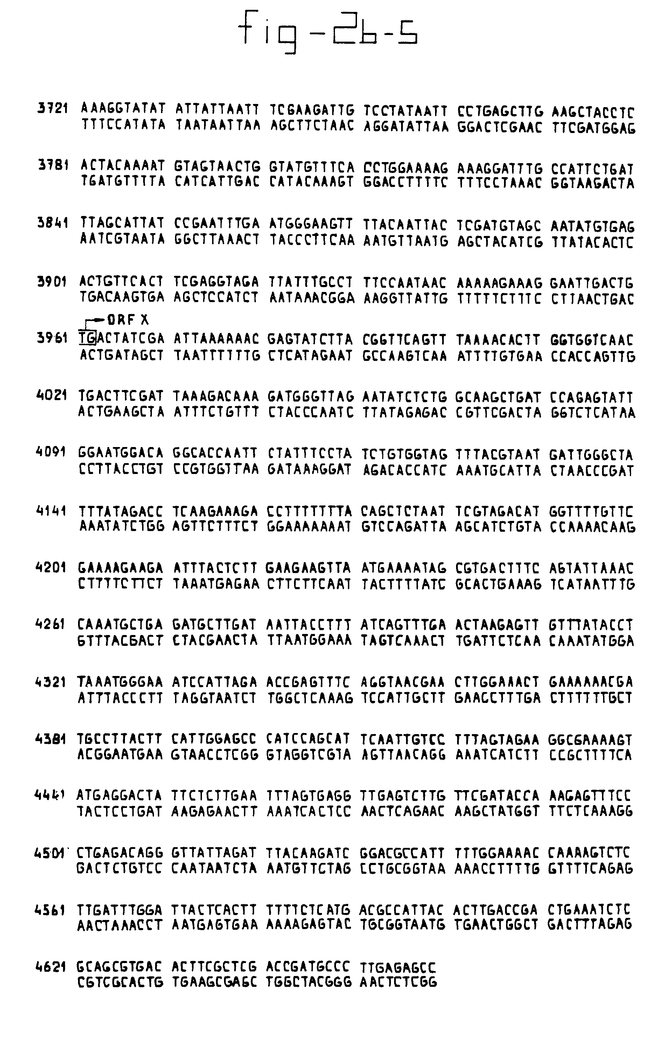

The plasmid pMG820 was cut with the restriction enzyme BstEII, after which the restriction fragments produced were blunted with Klenow polymerase. An approx. 4 kb fragment containing the complete Factor III gene and upstream regions was isolated and cloned in the HindII site of the E.coli vector pUC7. The resulting plasmid pNZ301 was used as a source for isolating the Factor III gene with as few flanking DNA sequences as possible. This was carried out by isolating the Factor III gene as a 0.4 kb NcoI-XmnI fragment (see Figure 2B) and after annealing it with T4 polymerase, cloning it in the HindII site of the E.coli vector pUC7. The Factor III gene was isolated from the resultant plasmid pNZ302 as a BamHI fragment and cloned in the BamHI site of the E.coli vector pUC18. Two orientations were obtained, designated pNZ303 and pNZ304. The Factor III gene was isolated from pNZ303 as a 0.4 kb XbaI-EcoRI fragment and ligated with the 4.8 kb XbaI-EcoRI fragment of the L.lactis vector pIL253 (Figure 3). The Factor III gene (0.4 kb) containing the XbaI-EcoRI fragment was likewise isolated from pNZ304 and ligated with the 4.8 kb XbaI-EcoRI fragment of pIL253. Both ligation mixtures were transformed to L.lactis Y2-5. From both the transformation with the ligation mixture containing pNZ303 DNA and the transformation with the ligation mixture containing pNZ304 DNA, EmR colonies were obtained which appeared to contain the desired plasmids having a size of 5.2 kb, pNZ305 and pNZ306 respectively, the restriction map of which is depicted in Figure 3. However, only pNZ305 appeared to complement the lactose deficiency of the host Y2-5. The explanation of this resides in the fact that the isolated factor III gene does not contain its own promoter and therefore has to rely on an external promoter which can be cloned in the correct orientation in front of the gene which is already present in the vector sector in the construct pNZ305. As a result, L.lactis containing pNZ305 is capable of forming in-gellow colonies on lactose indicator agar and wild-type growth on media which contain lactose as the only sugar.

L.lactis Y2-5 containing pNZ305 has been deposited with the Centraal Bureau voor Schimmelcultures (Central Office for Mould Cultures) in Baarn on 13 June 1988 under number CBS 416.88.

### Example III

Selection of S.lactis Y2-5 transformants containing pNZ305 without use of antibiotics.

S.lactis Y2-5 was transformed with 1 µg of pIL253, pNZ305 or no DNA, and lactose-proficient and/or erythromycin-resistant colonies were selected. The result shown in Table 1 indicates that comparable quantities of lac⁺ EmR and EmR transformants were obtained with pNZ305 DNA but not with the vector pIL253 DNA. Direct selection of transformants containing pNZ305 DNA also proved possible on LIA without an antibiotic. The background of lactose-proficient revertants (approx. 4 x 10² with no DNA or pIL253 DNA) presented no great problems in this connection because, of the 6 x 10² lac⁺ transformants obtained with pNZ305 DNA, 20% also proved to be EmR. Said lac⁺ transformants proved to contain the pNZ305 plasmid DNA as was to be expected. Selection of lac⁺ transformants on lactose indicator agar followed by a simple plasmid screening is thus adequate to obtain S.lactis Y2-5 transformants which contain the recombinant plasmid pNZ305 coding for, inter alia, Factor III, without using antibiotic-resistance markers.

### Example IV

Stability of S.lactis Y2-5 containing pNZ305.

An overnight culture was made from an isolated colony of S.lactis Y2-5 containing pNZ305 in LM17 medium containing 5 µg/ml Em. Through 1000 fold dilution hereof, three 10 ml cultures were prepared on LM17, WP and GLM 17. These were incubated at 30°C and diluted to 1000 every 10-14 hours in fresh medium of the same composition.

The quantity of lac⁺ and lac⁻ and also of lac⁺ EmR cells was regularly determined by plating out on LIA containing no or 5 µg/ml Em, or by streaking lac⁺ or lac⁻ colonies onto LIA containing 5 µg/ml Em. The result after 50 or 100 generations of growth in the various media indicates, as is shown in Table 2, that pNZ305 is unstable under non-selective conditions (glucose as sugar). The plasmid is stabilised, however, during growth under selective conditions (lactose as sugar), both in the LM17 broth and in the industrially applicable medium WP. All the lac⁺ cells furthermore prove to be EmR, from which it may be concluded that no gene conversion occurs from the chromosomal mutated Factor III gene to the plasmid-localised homologous copy, which would result in a lac⁺ phenotype and plasmid loss under non- selective conditions.

Finally, it is evident that the entire plasmid DNA is integrated in the chromosome if pNZ305 containing strain S.lactis Y2-5 is cultivated for 50 generations on a LM17 medium. Hybridisation experiments have indicated that in this case amplification to more than 25 copies of the entire plasmid DNA is achieved.

### Example V

Construction of a deficient pMG820 P-β-Gal gene.

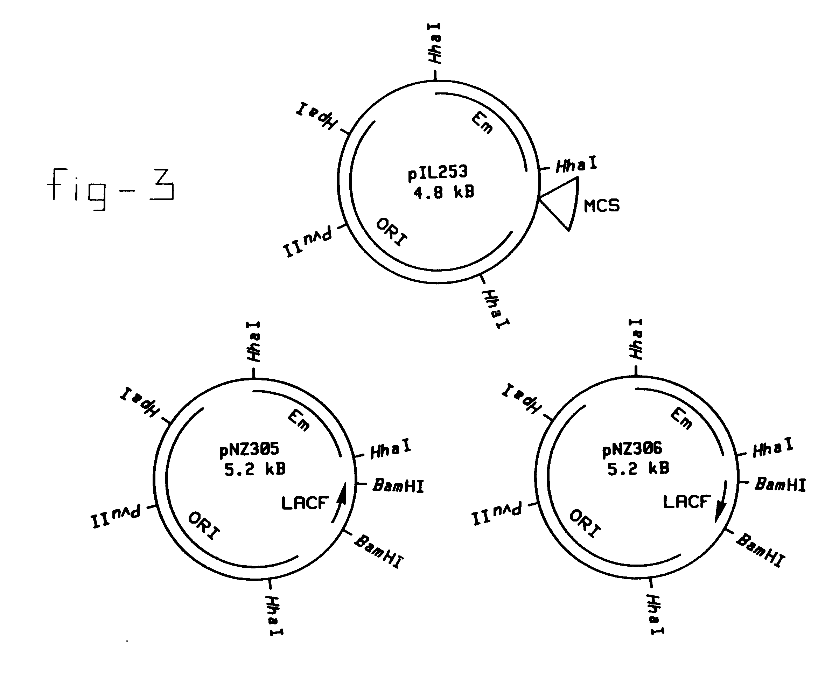

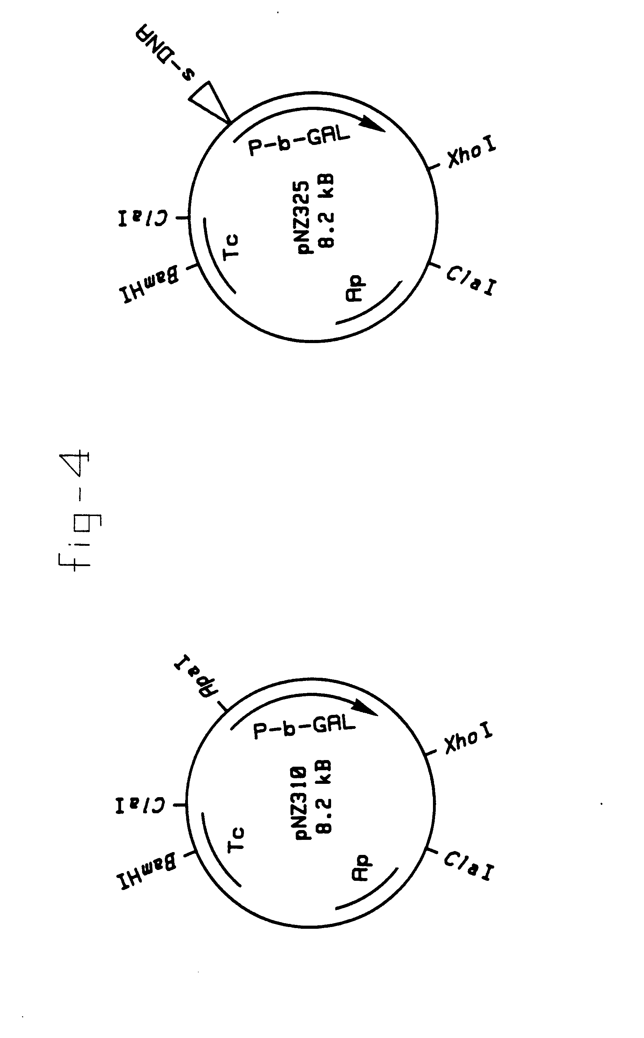

An approx. 4.6 kb ClaI (ClaC) fragment of pMG820 DNA was isolated and cloned in the E.coli vector PAT153. Of the two orientations which were obtained, pNZ310 and pNZ311, pNZ310 proved to have the highest specific P-β-Gal activity because, in this construct, the P-β-Gal gene is under the control of the anti-tet promotor of the vector. pNZ310 DNA was isolated and digested with ApaI, a unique site situated in the structural P-β-Gal gene (see Figure 4). In the thus linearised pNZ310 DNA, a 21 bp synthetic DNA fragment (s-DNA) was cloned which consisted of double-stranded DNA in which the base sequence of the individual strands is as follows: 5'-CGGGATCCGTCGACTAGGGCC-3' and 3'-CCGGGCCCTAGGCAGCTGATC-5'. Said s-DNA is chosen so that ApaI sites flank the fragment, as a result of which it can always be removed by digestion with said enzyme, and so that it contains unique SmaI, SalI and BamHI restriction sites and finally so that it interrupts the reading frame of the P-β-Gal gene with a TAG stop codon. The resultant plasmid pNZ325 provides no P-β-Gal activity in B.coli and contains the expected new SmaI, SalI and BamHI sites. In addition, an active P-β-Gal gene can be obtained by digesting pNZ325 with ApaI, followed by transformation of MC1061 with the purified linear pNZ325 DNA. From this it is evident that, starting from the particular nucleotide sequence of the lactose genes, it is simple to obtain in vitro an inactivated lactose gene with site-directed mutagenesis.

### CAPTIONS

Figure 1:Manner in which lactose is fermented by L.lactis to form lactate (according to FEMS Microbiol. Rev. 46 (1987), 221-231; Ant. van Leeuwenhoek 49 (1983), 209-224; Biochimie 70 (1988), Parts 3 and 4).
Figure 2A:Restriction map of pMG820 indicating the position of the lactose genes.
Figure 2B:Nucleotide sequence of those genes indicated in Figure 2A.
Figure 3:Restriction maps of pNZ305 and pNZ306 composed of the vector pIL253 and the L.lactis Factor III gene. MCS indicates the multiple cloning site of pIL253 which is composed of sites for, consecutively: ClaI, XbaI, XhoI, SacI, XhoI, XbaI, ClaI, HindIII, PstI, SalI, BamHI, SmaI, EcoRI. The MCS of pNZ305 and pNZ306 (not shown) mostly originates from pUC18 and is composed of ClaI-XbaI-BamHI-SmaI-KpnI-SacI-EcoRI sites, the Factor III gene being inserted in the unique BamHI site in the orientations indicated.
Figure 4:Restriction map of pNZ310 and pNZ325. s-DNA indicates the 21 bp synthetic DNA fragment containing the ApaI-SmaI-BamHI-SalI-ApaI sites and the TAG stop codon as described in the text.

**TABLE 1**

| DNA | TRANSFORMANTS per ml | | |
|---|---|---|---|
| | EmR | Lac⁺ | EmR Lac⁺ |
| none | < 101 | 4.0 x 10² | < 10¹ |
| pIL253 | 1,1 x 10² | 4.5 x 10² | < 10¹ |
| pNZ305 | 1.2 x 10² | 6.0 x 10² | 1.1 x 10² |
| Table 1: Transformation of S.lactis Y2-5 followed by selection of the gel lac⁺ and lac⁺ EmR transformants on LIA containing no or 5 µg/ml Em and EmR transformants on GM17 agar containing 5 µg/ml Em trans formants on LIA containing no or 5µg/ml Em. | | | |

**TABLE 2**

| Medium | Frequency of lac+ & EmR colonies | | | |
|---|---|---|---|---|
| | after 50 generations | | after 100 generations | |
| | Lac⁺ | EmR | Lac⁺ | EmR |
| LM17 | > 99% | > 99% | > 99% | > 99% |
| WP | > 99% | > 99% | > 99% | > 99% |
| GM17 | 35% | 35% | < 5% | < 5% |
| Table 2: Stability of S.lactis Y2-5 containing pNZ305 grown for 50 or 100 generations on media containing lactose (LM17, WP) or containing glucose (GM17). | | | | |

## Claims

1. Procedure for selecting lactic acid bacteria which contain recombinant DNA, and keeping said DNA stable, with the aid of a marker acceptable in foodstuffs for human consumption, **characterised in that**
a lactic acid bacterium which lacks one or two enzymes indispensable for lactose fermentation, chosen from Enzyme II, Factor III, p-β-galactosidase, gal-6-P-isomerase, tagatose-6-P-kinase, and/or tagatose-1,6-diP-aldolase;
is transformed with a DNA fragment which, as a marker,
- contains one or more genes coding at least for the said one or two enzymes lacking in said lactic acid bacterium and
- has the size of at most the genes coding for phospho-B-galactosidase and enzyme III,
and also contains one or more structural genes coding for an improved or new characteristic for the lactic acid bacterium.

2. Procedure according to Claim 1, **characterized in that** the lactic acid bacterium belongs to the *Lactococcus* or *Lactobacillus.*

3. Procedure according to Claims 1 - 2, **characterized in that** the lactic acid bacterium is a *Lactococcus lactis*.

4. Procedure according to Claims 1 - 3, **characterized in that** the lactic acid bacterium used is, as a result of a deletion, an insertion or point mutation in lactose genes which are situated on the chromosomal DNA, deficient in the synthesis of one or two of Enzyme II, Factor III, p-β-galactosidase, gal-6-P-isomerase, tagatose-6-P-kinase, and/or tagatose-1,6-diP-aldolase.

5. Procedure according to Claims 1 - 3, **characterized in that** the lactic acid bacterium used is, as a result of a deletion, an insertion or a point mutation in the lactose genes which are situated on plasmid DNA, deficient in the synthesis of one or two of Enzyme II, Factor III, p-β-galactosidase, gal-6-P-isomerase, tagatose-6-P-kinase, and/or tagatose-1,6-diP-aldolase.

6. Procedure according to Claims 1 - 4, **characterized in that** the lactic acid bacterium used is deficient in the synthesis of a lactose-specific Factor III and the DNA fragment at least codes for said Factor III.

7. Procedure according to Claims 1 - 3 and 5, **characterized in that** the lactic acid bacterium is deficient in the synthesis of a phospho-β-galactosidase and the DNA fragment at least codes for said phospho-β-galactosidase.

8. Procedure according to Claim 7, **characterized in that** the DNA fragment also codes for the production of proteolytic enzymes such as proteinase(s) or peptidase(s), codes for insensitivity to bacteriophages, codes against decomposition of citrate, codes for bacteriocin production and/or bacteriocin immunity or codes for β-galactosidase, α-amylase, chymosin or pepsin.

9. Procedure according to Claim 7 or 8, **characterized in that** the DNA fragment also contains a replicon which is functional in the lactic acid bacterium used.

10. Procedure according to Claims 7 - 9, **characterized in that** the DNA fragment also contains a DNA sequence which is homologous with the DNA of the lactic acid bacterium used, as a result of which the entire DNA fragment or essential parts thereof is or are integrated into the DNA of said lactic acid bacterium and possibly amplified.

11. Procedure according to any of the preceding claims, in which the DNA fragment used as a marker is derived from DNA from a lactic acid bacterium.

12. Procedure according to any of the preceding claims, in which the DNA fragment is in the form of a cloning, expression, secretion or integration vector.

13. Procedure according to any of the preceding claims, which further comprises culturing/maintaining the lactic acid bacteria in a medium comprising lactose as essentially the only fermentable sugar in the culture medium.

14. *L.lactis* Y2-5 containing pNZ305 deposited with the Centraal Bureau voor Schimmelcultures in Baarn, The Netherlands, under number CBS 416.88.

## Patentansprüche

1. Verfahren zur Selektion von Milchsäurebakterien, die rekombinante DNA enthalten, wobei die DNA mit Hilfe eines Markers, der in Nahrungsmitteln für den menschlichen Verzehr akzeptabel ist, stabilisiert wird, **dadurch gekennzeichnet, dass** ein Milchsäurebakterium, dem ein oder zwei für die Lactosefermentation unerlässliche Enzyme fehlen, die aus Enzym II, Faktor III, p-β-Galactosidase, Gal-P-Isomerase, Tagatose-6-P-Kinase und/oder Tagatose-1,6-diP-Aldolase gewählt sind, mit einem DNA-Fragment transformiert wird, das als Marker
- ein oder mehrere Gene enthält, die mindestens für das eine oder die zwei Enzyme, die im Milchsäurebakterium fehlen, codieren und
- eine Größe aufweist, die höchstens den Genen entspricht, die für Phospho-β-Galactosidase und Enzym III codieren
und ebenfalls ein oder mehr Strukturgene enthält, die für eine verbesserte oder neue Eigenschaft des Milchsäurebakteriums codieren.

2. Verfahren nach Anspruch 1, **dadurch gekennzeichnet, dass** das Milchsäurebakterium zum Lactococcus oder Lactobacillus gehört.

3. Verfahren nach den Ansprüchen 1 bis 2, **dadurch gekennzeichnet, dass** das Milchsäurebakterium Lactococcus lactis ist.

4. Verfahren nach den Ansprüchen 1 bis 3, **dadurch gekennzeichnet, dass** das verwendete Bakterium ein Ergebnis einer Deletion, einer Insertion oder einer Punktmutation in den Lactogenen ist, die sich auf der chromosomalen DNA befinden und defizient in der Synthese von einem oder zwei der Enzyme Enzym II, Faktor III, p-β-Galactosidase, Gal-6-P-Isomerase, Tagatose-6-P-Kinase und/oder Tagatose-1,6-diP-Aldolase sind.

5. Verfahren nach den Ansprüchen 1 bis 3, **dadurch gekennzeichnet, dass** das verwendete Milchsäurebakterium ein Ergebnis einer Deletion, einer Insertion oder einer Punktmutation in den Lactosegenen ist, die sich auf Plasmid-DNA befinden und defizient in der Synthese von einem oder zwei der Enzyme Enzym II, Faktor III, p-β-Galactosidase, Gal-6-P-Isomerase, Tagatose-6-P-Kinase und/oder Tagatose-1,6-diP-Aldolase sind.

6. Verfahren nach den Ansprüchen 1 bis 4, **dadurch gekennzeichnet, dass** das verwendete Milchsäurebakterium in der Synthese eines lactosespezifischen Faktors III defizient ist und das DNA-Fragment mindestens für diesen Faktor III codiert.

7. Verfahren nach den Ansprüchen 1 bis 3 und 5, **dadurch gekennzeichnet, dass** das Milchsäurebakterium in der Synthese einer Phospho-β-Galactosidase defizient ist und das DNA-Fragment mindestens für diese Phospho-β-Galactosidase codiert.

8. Verfahren nach Anspruch 7, **dadurch gekennzeichnet, dass** das DNA-Fragment ebenfalls für die Herstellung von proteolytischen Enzymen, wie (die) Proteinase(n) oder Peptidase(n), codiert, für die Unempfindlichkeit gegenüber Bakteriophagen codiert, gegen die Zersetzung von Citrat codiert, für die Bakteriocinproduktion und/oder Bakteriocinimmunität codiert oder für β-Galactosidase, α-Amylase, Chymosin oder Pepsin codiert.

9. Verfahren nach Anspruch 7 oder 8, **dadurch gekennzeichnet, dass** das DNA-Fragment ebenfalls ein Replikon enthält, das im verwendeten Milchsäurebakterium funktional ist.

10. Verfahren nach den Ansprüchen 7 bis 9, **dadurch gekennzeichnet, dass** das DNA-Fragment ebenfalls eine DNA-Sequenz enthält, die zu der DNA des verwendeten Milchsäurebakteriums homolog ist und im Ergebnis das gesamte DNA-Fragment oder wesentliche Teile davon in der DNA dieses Milchsäurebakteriums integriert und möglicherweise amplifiziert ist oder sind.

11. Verfahren nach einem der vorangegangenen Ansprüche, worin das als Marker verwendete DNA-Fragment von DNA aus einem Milchsäurebakterium abgeleitet ist.

12. Verfahren nach einem der vorangegangenen Ansprüche, worin das DNA-Fragment in Form eines Klonierungs-, Expressions-, Sekretions- oder Integrationsvektors vorliegt.

13. Verfahren nach einem der vorangegangenen Ansprüche, worin weiterhin die Milchsäurebakterien in einem Medium, das Lactose als im Wesentlichen den einzigen fermentierbaren Zucker. im Kulturmedium enthält, gezüchtet/gehalten werden.

14. L.lactis Y2-5, der pNZ305 enthält und im Central Bureau for Schimmelcultures in Baarn, Niederlande unter der Nummer CBS 416.88 hinterlegt ist.

## Revendications

1. Procédé pour sélectionner des bactéries lactiques qui contiennent de l'ADN recombiné et maintenir cet ADN stable, à l'aide d'un marqueur acceptable dans les produits alimentaires destinés à la consommation humaine,
**caractérisé en ce que**
une bactérie lactique à laquelle manque une ou deux enzymes indispensables à la fermentation du lactose, choisies parmi l'Enzyme II, le Facteur III, la p-β-galactosidase, la gal-6-P-isomérase, la tagatose-6-P-kinase et/ou la tagatose-1,6-diP-aldolase, est transformée avec un fragment d'ADN qui, en tant que marqueur,
- contient un ou plusieurs gènes codant au moins pour ladite une ou lesdites deux enzymes manquantes dans ladite bactérie lactique et
- a au maximum la taille des gènes codant pour la phospho-β-galactosidase et l'enzyme III,
et contient également un ou plusieurs gènes de structure codant pour une caractéristique améliorée ou nouvelle de ladite bactérie lactique.

2. Procédé selon la revendication 1, **caractérisé en ce que** la bactérie lactique appartient au genre *Lactococcus* ou *Lactobacillus.*

3. Procédé selon les revendications 1 et 2, **caractérisé en ce que** la bactérie lactique est *Lactococcus lactis.*

4. Procédé selon les revendications 1 à 3, **caractérisé en ce que** la bactérie lactique utilisée est, en conséquence d'une délétion, d'une insertion ou d'une mutation ponctuelle dans les gènes de lactose qui sont situés sur l'ADN chromosomique, déficiente dans la synthèse d'un ou deux de l'Enzyme II, du Facteur III, de la p-β-galactosidase, de la gal-6-P-isomérase, de la tagatose-6-P-kinase et/ou de la tagatose-1,6-diP-aldolase.

5. Procédé selon les revendications 1 à 3, **caractérisé en ce que** la bactérie lactique utilisée est, en conséquence d'une délétion, d'une insertion ou d'une mutation ponctuelle dans les gènes de lactose qui sont situés sur un ADN plasmidique, déficiente dans la synthèse d'un ou deux de l'Enzyme II, du Facteur III, de la p-β-galactosidase, de la gal-6-P-isomérase, de la tagatose-6-P-kinase et/ou de la tagatose-1,6-diP-aldolase.

6. Procédé selon les revendications 1 à 4, **caractérisé en ce que** la bactérie lactique utilisée est déficiente dans la synthèse d'un Facteur III spécifique du lactose et le fragment d'ADN code au moins pour ledit Facteur III.

7. Procédé selon les revendications 1 à 3 et 5, **caractérisé en ce que** la bactérie lactique est déficiente dans la synthèse d'une phospho-β-galactosidase et le fragment d'ADN code au moins pour ladite phospho-β-galactosidase.

8. Procédé selon la revendication 7, **caractérisé en ce que** le fragment d'ADN code également pour la production d'enzymes protéolytiques telles qu'une ou plusieurs protéinase(s) ou peptidase(s), code pour l'insensibilité aux bactériophages, code contre la décomposition de citrate, code pour la production de bactériocine et/ou l'immunité à une bactériocine ou code pour la β-galactosidase, la α-amylase, la chymosine ou la pepsine.

9. Procédé selon la revendication 7 ou 8, **caractérisé en ce que** le fragment d'ADN contient également un réplicon qui est fonctionnel dans la bactérie lactique utilisée.

10. Procédé selon les revendications 7 à 9, **caractérisé en ce que** le fragment d'ADN contient également une séquence d'ADN qui est homologue à l'ADN de la bactérie lactique utilisée, en conséquence de quoi le fragment d'ADN entier ou ses parties essentielles est ou sont intégrés dans l'ADN de ladite bactérie lactique et éventuellement amplifiés.

11. Procédé selon l'une quelconque des revendications précédentes, dans laquelle le fragment d'ADN utilisé comme marqueur est dérivé d'ADN provenant d'une bactérie lactique.

12. Procédé selon l'une quelconque des revendications précédentes, dans laquelle le fragment d'ADN est sous la forme d'un vecteur de clonage, d'expression, de sécrétion ou d'intégration.

13. Procédé selon l'une quelconque des revendications précédentes, qui comprend de plus la culture/l'entretien des bactéries lactiques dans un milieu comprenant du lactose comme essentiellement seul sucre fermentable dans le milieu de culture.

14. *L*. *lactis* Y2-5 contenant pNZ305 déposé au Centraal Bureau voor Schimmelcultures de Baarn, Pays-Bas, sous le numéro CBS 416.88.
